# EUROPEAN PATENT SPECIFICATION

(11) **EP 1 893 640 B1**
(45) Date of publication and mention of the grant of the patent: **01.04.2015**
(21) Application number: 06721644.0
(22) Date of filing: 03.05.2006
(51) Int. Cl.: C07K 14/435, C07K 4/12, A61P 33/00

(54) **COMPOSITION COMPRISING FUCOSE MANNOSE LIGAND (FML) AND SAPONIN FOR PREPARATION OF LEISHMANIASIS TRANSMISSION BLOCKING VACCINES IN HUMANS AND ANIMALS**
ZUSAMMENSETZUNG AUS FUCOSE MANNOSE LIGAND (FML) UND SAPONIN ZUR HEMMUNG DER ÜBERTRAGUNG VON LEISHMANIASIS BEI MENSCHEN UND TIEREN
COMPOSITION CONTENANT LE LIGAND FUCOSE-MANNOSE (FML) ET DE LA SAPONINE BLOQUANT LA TRANSMISSION DE LA LEISHMANIOSE CHEZ L'HOMME ET CHEZ L'ANIMAL

(30) Priority: 16.05.2005 BR PI0503187
(43) Date of publication of application: 05.03.2008
(73) Proprietor: Universidade Federal Do Rio De Janeiro - UFRJ, 20.530-310 Ilha do Governador, Rio de Janeiro (BR)
(72) Inventor: PALATNIK DE SOUZA, Clarisa B., Rio de Janeiro - RJ (BR); CHEQUER BOU-HABIB, Elvira Maria Saraiva, Rio de Janeiro - RJ (BR)
(74) Representative: PROPI, S.L.
(86) International application number: PCT/BR2006/000086
(87) International publication number: WO 2006/122382

(56) References cited:
- BR-A- 9 302 386
- BORJA-CABRERA G P ET AL: "Effective immunotherapy against canine visceral leishmaniasis with the FML-vaccine" VACCINE, BUTTERWORTH SCIENTIFIC. GUILDFORD, GB, vol. 22, no. 17-18, 2 June 2004 (2004-06-02), pages 2234-2243, XP004508741 ISSN: 0264-410X
- SANTOS W R ET AL: "Immunotherapy against murine experimental visceral leishmaniasis with the FML-vaccine" VACCINE, BUTTERWORTH SCIENTIFIC. GUILDFORD, GB, vol. 21, no. 32, 1 December 2003 (2003-12-01), pages 4668-4676, XP004469683 ISSN: 0264-410X
- SANTOS W R ET AL: "Saponins, IL12 and BCG adjuvant in the FML-vaccine formulation against murine visceral leishmaniasis" VACCINE, BUTTERWORTH SCIENTIFIC. GUILDFORD, GB, vol. 21, no. 1-2, 22 November 2002 (2002-11-22), pages 30-43, XP004393283 ISSN: 0264-410X
- BORJA-CABRERA G P ET AL: "Long lasting protection against canine kala-azar using the FML-QuilA saponin vaccine in an endemic area of Brazil (Sao Goncalo do Amarante, RN)" VACCINE, BUTTERWORTH SCIENTIFIC. GUILDFORD, GB, vol. 20, no. 27-28, 10 September 2002 (2002-09-10), pages 3277-3284, XP004378522 ISSN: 0264-410X
- COLER R N ET AL: "Second-generation vaccines against leishmaniasis" TRENDS IN PARASITOLOGY, ELSEVIER CURRENT TRENDS, vol. 21, no. 5, 18 March 2005 (2005-03-18), - May 2005 (2005-05) pages 244-249, XP004855970 ISSN: 1471-4922
- DA SILVA V O ET AL: "A phase III trial of efficacy of the FML-vaccine against canine kala-azar in an endemic area of Brazil (Sao Goncalo do Amaranto, RN)" VACCINE, BUTTERWORTH SCIENTIFIC. GUILDFORD, GB, vol. 19, no. 9-10, 8 December 2000 (2000-12-08), pages 1082-1092, XP004227313 ISSN: 0264-410X
- PALATNIK C B ET AL: "Inhibition of Leishmania donovani promastigote internalization into murine macrophages by chemically defined parasite glycoconjugate ligands." INFECTION AND IMMUNITY MAR 1989, vol. 57, no. 3, March 1989 (1989-03), pages 754-763, XP002492637 ISSN: 0019-9567
- LEMESRE J-L ET AL: "Protection against experimental visceral leishmaniasis infection in dogs immunized with purified excreted secreted antigens of Leishmania infantum promastigotes" VACCINE, BUTTERWORTH SCIENTIFIC. GUILDFORD, GB, vol. 23, no. 22, 22 April 2005 (2005-04-22), pages 2825-2840, XP004797054 ISSN: 0264-410X
- DYE C: "The logic of visceral leishmaniasis control." THE AMERICAN JOURNAL OF TROPICAL MEDICINE AND HYGIENE AUG 1996, vol. 55, no. 2, August 1996 (1996-08), pages 125-130, XP009104688 ISSN: 0002-9637
- BORJA-CCABRERA ET AL.: 'Long lasting protection against canine kala-azar using the FML-Quil A saponin vaccine in an endemic area of Brazin (Sao Goncalo do Amarante, RN)' VACCINE vol. 20, 10 September 2002, pages 3277 - 3284, XP004378522
- DA SILVA ET AL.: 'A Phase III trial of efficacy of the PML-vaccine against canine kala-azar in an endemic area of Brazil (Sao Boncalo do Amaranto, RN' VACCINE vol. 19, 2000, pages 1082 - 1092, XP004227313
- 'Leishmune: Manual tecnico', [Online] Retrieved from the Internet: <URL:http://www.leishmune.com.br/download/m anual/manual.leishmune.pdf> [retrieved on 2008-08-19]
- SARAIVA E M ET AL: "The FML-vaccine (Leishmune<(>R)) against canine visceral leishmaniasis: A transmission blocking vaccine", VACCINE, ELSEVIER LTD, GB, vol. 24, no. 13, 20 March 2006 (2006-03-20), pages 2423-2431, XP028011210, ISSN: 0264-410X, DOI: 10.1016/J.VACCINE.2005.11.061 [retrieved on 2006-03-20]
- PALATNIK-DE-SOUSA CLARISA B ET AL: "FML vaccine against canine visceral leishmaniasis: from second-generation to synthetic vaccine", EXPERT REVIEW OF VACCINES ENGLAND MAR 2009, EXPERT REVIEW OF VACCINES ENGLAND MAR 2009, vol. 7, no. 6, 1 August 2008 (2008-08-01), pages 833-851, XP009125352, ISSN: 1744-8395, DOI: 10.1586/14760584.7.6.833
- OLIVEIRA-FREITAS E ET AL: "Acylated and deacylated saponins of Quillaja saponaria mixture as adjuvants for the FML-vaccine against visceral leishmaniasis", VACCINE, ELSEVIER LTD, GB, vol. 24, no. 18, 1 May 2006 (2006-05-01), pages 3909-3920, XP028010856, ISSN: 0264-410X, DOI: 10.1016/J.VACCINE.2006.02.034 [retrieved on 2006-05-01]
- NOGUEIRA F S ET AL: "Leishmune<(>R) vaccine blocks the transmission of canine visceral leishmaniasis", VACCINE, ELSEVIER LTD, GB, vol. 23, no. 40, 23 September 2005 (2005-09-23), pages 4805-4810, XP027652045, ISSN: 0264-410X [retrieved on 2005-09-23]

## Description

### Field of invention

This invention refers to pharmaceutical compositions suitable for preparation of leishmaniasis transmission blocking vaccines in humans and animals from Leishmania promastigotes or Leishmania amastigotes fractions (FML). Such composition, when administered in combination with a saponin adjuvant, blocks the visceral Leishmaniasis transmission in humans and animals.

### Background of the invention

In America and Mediterranean, the human visceral Leishmaniasis, or kala-azar, is a canine zoonose. *Phlebotominae* insects when feeding from wild canideous acquire the ethiologic agent, transmitting it later to dogs. The subsequent transmission to humans by *Phlebotominae* insects causes visceral human Leishmaniasis, a serious disease that can be fatal if not adequately treated.

Approximately 1,000,000 new human occurrences of kala-azar are registered annually in the world. A protective vaccine for prophylaxis against human disease is not yet available. The best performance was achieved with a first generation experimental vaccine which induced 12% of protection, just among people that converted to positive in intradermal tests for Leishmania lysate after complete vaccination. Even today, the chemotherapy against kala-azar is highly toxicant and not always effective.

Those vectors are a canine zoonose transmitted by insects and they present infection reduction through ingestion of anti-Leishmania antibodies present in serum of dogs submitted to protective vaccination having great impact for reduction of the disease incidence in humans.

The brazilian patent PI9302386 describes a product called FML antigen, "Ligante Fucose-Manose" or "Fucose Mannose Ligand" for *Leishmania donovani* comprising a glycoprotein complex whose carbohydrate portion presents 10% of Fucose and 47% of Manose, justifying the utilized name. This complex strongly inhibits mouse peritoneal macrofags infection due *L. donovani* promastigotes and amastigotes, it being species-specific in the Leishmania gender. FML is strongly immunogenic in rabbits, hamsters and mice stimulating intense antibodies production. It is antigenic in humans and is recognized by high levels of specific antibodies.

The FML initial biochemical analysis has revealed that FML is a glycoprotein compound containing 29% of neutral sugar, 44% of protein, 11% of phosphate and hexamine traces composed by protein bands with molecular weights ranging from 9 to 95 kDa, some protein bands having carbohydrates: 36 and 54 to 68 kDa. The FML glycidic portion stands out the presence of 10% of fucose and 47% of manose. It was also possible to observe the presence of 30% of glucose, 12% of galactose and xylose in trace-quantity.

The FML N-linked oligosaccharides form part of a ramified chain alternating units of Manp 4-*O*, 3-*O*, Glc Nac 4-*O-*linked; N-acetil-glucosamine as a ramification point and fucopyranose, galactopyranose and manopyranose as terminal residues, or linear oligosaccharides composed by units of Manp 4-*O*, 3-*O* and 2-*O*-linked, 2-*O*-linked, fucopyranose and galactopyranose as terminal residues (Thesis presented at UFRJ in April 14, 1998 by Robson R. Bernardo).

The FML aminoacid analysis has revealed presence of glutamic acid, alanine, aspartic acid and glycine in quantities ranging from 20 to 10%, serine, histidine, lisin, leucine, threonine, proline and valine ranging from 5 and 10%, and arginine, methionine, isoleucine, tyrosine and phenylalanine ranging from 2 and 5%.

The Master's Degree Dissertation "Estudos sobre a glicoproteina GP36 de e sobre o eu potencial na vacinação contra o calazar experimental" ("GP36 glycoprotein studies and its potential in vaccination against experimental kala-azar") presented at UFRJ by Edilma Paraguai de Souza identified a glycoprotein having molecular weight of 36 kDa as a FML predominant antigen, called GP36. *Leishmania donovani* studies by C.B. Palatnik de Sousa, H.S. Dutra, and R. Borojevic, in Acta Tropica, 53:59-72 confirm that GP36 is recognized by the hiper-immune rabbit serum produced against total FML. Monoclonal antibodies obtained against FML recognize predominantly GP36. The GP36 antigenicity was susceptible to sodium periodate treatment indicating the glycidic nature of its epitopes involved in the reaction with monoclonal antibodies, (ACE10 antibody), therefore confirming its glycoprotein nature. Furthermore, the Master's Degree Dissertation presented at UFRJ in March 25, 1998 by Edilma P. de Souza shows that this fraction contains, among the aminoacids, from 10 to 20% of aspartic acid, glutamic acid, alanine and leucine, from 5 to 10% of glycine, threonine, valine and lisin, from 5 to 2% of serine, histidine, arginine, proline, tyrosine, methionine, isoleucine and phenylalanine and that its N-linked oligosaccharides revealed fucose residues with two substitution types (2,3-Me₂-Fucose, 2,4-Me₂-Fucose) and a predominance of manopyranose residues of 2,3,6,-Me₃-Manose type, besides tri-Me₃-Galactose corresponding to 4-*O*-substituted manopyranose linear chains alternating with substituted 3-*O* and 4-*O*, fucopyranose residues.

The above mentioned R. Acta Tropica also reveals that does not exist crossed reactivity between FML and glycoprotein 63, GP63, previously isolated from Leishmania. Specific monoclonal antibodies L-3.2 and L-3.9 obtained by David Russell against GP63 do not develop any reaction to FML, nor in Western Blotting assay, nor in ELISA assay, the latter considered the most sensitive. This result is coherent and previsible, considering the different extraction methods. The aqueous extraction of FML, including double heating to 100°C, privileges the obtaining of glycoconjugates whose glycidic fraction is resistant to these conditions.

The GP36 protein portion was recently cloned in the *E. coli.* system mentioned in "Molecular and Biochemical Parasitology", 120:315-319. It is a nucleosidide hydrolase of 34 kDa *L. (L.) donovani* having 75% of identity with Inosine Uridine Hydrolase (IUNH) of *Crithidia fasciculata* and 90% of homology with rNH (IUNH) of *L. (L. major).* The nucleoside hydrolase of *L. (L.) donovani* is a specific diagnosis marker for canine kala-azar, as described in this publication. The sequence of bases and aminoacidics of the nucleoside hydrolase of *L. (L.) donovani,* described in the Thesis of Doctorate "Clonagem molecular do antigeno GP36 de *L. donovani"* ("Molecular cloning of L. donovani antigen GP36") presented at UFRJ in May 14, 2002, is represented below:

| |
|---|
| ATGCCGCGCAAGATTATTCTCGATTGTGATCCCGGGATCGATGATGCCGTGGCCATCTTTCTCGC CCACGGCAACCCGGAGGTCGAG 87 |
| M P R K I I L D C D P G I D D A V A I F L A H G N P E V E |
| CTGCTGGCCATTACGACGGTGGTGGGCAACCAGACCCTGGAGAAGGTGACCCGGAACGCGCGG CTGGTAGCTGACGTAGCCGGCATC 174 |
| L L A I T T V V G N Q T L E K V T R N A R L V A D V A G I |
| GTTGGTGTGCCCGTCGCGGCTGGTTGCACCAAGCCCCTCGTGCGCGGTGTGCGGAATGCCTCTC AGATTCATGGCGAAACCGGCATG 261 |
| V G V P V A A G C T K P L V R G V R N A S Q I H G E T G M |
| GGTAACGTCTCCTACCCACCAGAGTTCAAGACAAAGTTGGACGGCCGTCATGCAGTGCAGCTGA TCATCGACCTTATCATGTCGCAC 348 |
| GGTAACGTCTCCTACCCACCAGAGTTCAAGACAAAGTTGGACGGCCGTCATGCAGTGCAGCTGA TCATCGACCTTATCATGTCGCAC 348 |
| G N V S Y P P E F K T K L D G R H A V Q L I I D L I M S H |
| GAGCCGAAGACGATCACGCTTGTGCCTACGGGTGGCCTGACGAACATTGCGATGGCTGTCCGTC TTGAGCCGCGCATCGTGGACCGT 435 |
| E P K T I T L V P T G G L T N I A M A V R L E P R I V D R |
| GTGAAGGAGGTGGTTCTGATGGGTGGCGGCTACCATACTGGTAATGCGTCCCCTGTAGCGGAGT TCAACGTCTTCGTCGACCCGGAG 522 |
| V K E V V L M G G G Y H T G N A S P V A E F N V F V D P E |
| GCGGCGCACATTGTGTTCAACGAGAGCTGGAACGTAACGATGGTGGGGCTGGACCTAACGCAC CAGGCACTCGCCACGCCGGCGGTC 609 |
| A A H I V F N E S W N V T M V G L D L T H Q A L A T P A V |
| CAGAAGCGAGTGAAGGAGGTGGGCACGAAGCCGGCTGCCTTCATGCTGCAGATTTTGGACTTTT ACACGAAGGTGTACGAAAAGGAG 696 |
| Q K R V K **E V G T K P A A F M L Q I L D F Y T K** V Y E K E |
| CGCAACACGTACGCGACGGTGCACGATCCCTGCGCTGTGGCGTACGTGATTGACCCCACCGTGA TGACGACGGAGCAAGTGCCAGTG 783 |
| R N T Y A T V H D P C A V A Y V I D P T V M T T E Q V P V |
| GACATCGAGCTCAATGGGGCACTGACGACTGGGATGACGGTCGCGGACTTCCGCTACCCACGGC CAAAGCACTGCCACACGCAGGTG 870 |
| D I E L N G A L T T G M T V A D F R Y P R P K H C H T Q V |
| GCTGTGAAGCTGGACTTCGACAAGTTTTGGTGCCTCGTGATTGACGCACTCAAGCGCATCGGCG ATCCTCAATGA 945 |
| A V K L D F D K F W C L V I D A L K R I G D P Q |

A second FML antigenic component is a 55 kDa glycoprotein recognized by 7 IgM type monoclonal antibodies and only one of the IgG type, as mentioned in R. Acta Tropica.

### Description of the invention

The present invention describes the development of a prophylaxis vaccine against canine visceral leishmaniasis based on *L. donovani* saponin and FML antigen (Fucose Mannose ligand). The FML vaccine developed 92-95% of specific protection (76-80% of vaccine effectiveness) in Phase III assays against brazilian visceral Leishmaniasis, according to studies described in Vaccine 19:1082-1092, 2001, and Vaccine 20:3277-3284, 2002. These studies showed that vaccination has reduced the mortality and incidence of the canine disease. The research published in Vaccine 22 (17-18):2234-2243, 2004 mentioned the immunotherapeutic effect of the vaccine.

The term Transmission Blocking Vaccine (TBV) is used for anti-malaria vaccines that stimulate the antibodies production in humans against the sexual forms (gametes) of parasites present in the medium intestine of the *Anopheles* vector. Mosquitos acquire those antibodies during blood feeding, blocking the fertilization process and subsequent development of the parasites in the medium intestine of the vector, impeding the disease transmission by the insect.

Thus, the TBV is developed to increase the antibodies against the gamete-phase of malaria parasite present in the mosquito intestine and in spite of not eliminating the disease in the infected human, it prevents the malaria propagation among the community.

The mosquito acquires the antibodies during blood feeding and they would block the parasite development turning the vector in a no-infectious vector. In case of malaria, the antigens that are candidates to TBV are cellular surface antigens present in the gamete-phase of the microorganism. A functional test is applied to the analysis of the Phase I of this vaccine type. Mosquitos experimentally created in laboratory and previously infected are fed with hiper-immune serum against the subject antigen. Later the intestine of the mosquitos can be dissected to determine the number of infectious gametocytes.

In the epidemic cycle of visceral leishmaniasis agents, *Leishmania chagasi* and *Leishmania infantum,* the *Phlebotominae,* during blood meal, ingests macrofags and monocytes containing amastigotes of infected dogs. Those amastigotes delivered in the medium intestine of the vector differ in flagellated and procyclical promastigotes linked to the medium intestine epithelium. The procyclical promastigotes become divided by metacyclogenesis acquiring virulence and they become metacyclic promastigotes non-divided that are separated from the medium intestine epithelium migrating to the buccal cavity and infecting new vertebrates during the next blood meal.

The FML antigen is a surface antigen of *L*. *donovani* promastigotes and amastigotes, highly immunogenic and protective in the canine vaccination, so it could also be recognized by specific receivers in the medium intestine of vectors as *Lutzomyia longipalpis,* acting as a parasite ligand for the medium intestine membrane of the same.

In the present invention, assays were made relative to the FML presence for specific receivers and to the growing capacity of the antibodies in dogs after vaccination with Leishmune® (FML vaccine under industrialized license) for blocking the adhesion of *Leishmania donovani* and *Leishmania chagasi* procyclical promastigotes to the membrane of the medium intestine of the vector, the "sandfly" *Lutzomyia longipalpis,* making possible to characterize the potential blocking effect for the FML vaccine.

The illustrations A, B, C and D are part of the present application presenting the binding inhibition percentage, in percentage versus FML concentration in µg/ml or anti-FML Fab antibodies in µg/ml for *L. donovani* and *L. chagasi.*

The present invention relates to a Pharmaceutical Composition having Leishmania promastigotes or amastigotes fractions called Fucose Mannose Ligand (FML) and saponin.

Advantageously, the invention comprises a pharmaceutical composition for preparation of a leishmaniasis transmission blocking vaccine in humans and animals having Leishmania promastigotes or amastigotes fractions called FML.

More advantageously, the invention refers to a Pharmaceutical Composition having Leishmaniasis amastigotes or promastigotes fractions called Fucose Mannose Ligand (FML) comprising native compounds containing from 10 to 20% of glutamic acid, alanine, aspartic acid and glycine, from 5 to 10% of serine, histidine, lisin, threonine, proline and valine and from 2 to 5% of arginine, methionine, isoleucine, tyrosine and phenylalanine.

The invention comprises preferentially the use of the Pharmaceutical Composition in the preparation of a blocking vaccine to impede the Leishmaniasis transmission in humans and animals.

More preferentially, the invention comprises the use of the Pharmaceutical Composition in the preparation of a blocking vaccine to impede the Leishmaniasis transmission consisting in administration of Leishmania promastigotes or amastigotes fractions called saponin-(FML).

The invention will be better understood from the following assay having a merely exemplary and non-limitating scope.

Four healthy adult dogs without defined race were vaccinated with Leishmune (licentiated industrialized vaccine). The animals received three doses of the vaccine, with an interval of 20 days between each dose, through subcutaneous way. The serums were collected 20 days after the third application. The titles of the IgG type anti-FML antibodies were determined using the FML-ELISA test. The IgG absorbence to 492 nm in 1/100 diluted serums were: 0.818; 0.761; 0.887 and 0.776. All serums presented higher absorbence levels in the IgG2 than IgG1 subtype. To purify the IgG samples, all samples were diluted, precipitated and incubated by agitation. The precipitate was separated by centrifugation, resuspended and dialised. The isolated fraction was applied in a Protein-to-Sepharose column. The non-linked fraction was eluted, while the IgG purified fraction was recovered, neutralized and again dialised in buffer.

The protein concentration was determined and IgG purified was concentrated against polyethylene glycol. The Fab and Fc IgG fragments were obtained using an Immuno Pure Fab kit and subsequent chromatography in a Protein-to-Sepharose column. The Fab fragments were eluted in the first 2 volumes of the column while the Fc fraction was removed using a specific elution buffer.

Initially, the promastigotes binding to the medium intestine of the vectors was inhibited in the presence of FML. For such case, intestinal membranes of *Lutzomyia longipalpis* were dissected, longitudinally cut, pré-incubated with FML (40, 100, 200 and 400 µg/ml) and later they were incubated with 10⁶ procyclical promastigotes.

The assays demonstrated the presence of FML receivers on the membranes surface of the medium intestine of *Phlebotominae.* Such receivers recognized the FML compound on the surface of *L*. *donovani* or *L. chagasi* promastigotes phases acting as a parasite ligand for the medium intestine membrane. The medium intestine membranes pre-incubation with increased concentrations of FML then would inhibit the subsequent binding of the promastigotes phases. The results are summarized in the illustrations A and B. The FML concentration increase determines the binding inhibition increase for *L. donovani* and *L.chagasi.*

All FML concentrations induced significantly higher binding inhibition levels than saline control. Similarly, inhibition differences were observed among different FML concentrations, except 100 and 200 µg/ml for *L. donovani.*

Although the *L. donovani* isolated FML antigen has inhibited the binding of both parasites, according to illustrations A and B, it was observed that the inhibition was more pronounced against *L. donovani* promastigotes than against L. *chagasi* indicating the species-specific character of the homologs antigens recognition.

Binding inhibition assays were executed by pré-incubation of the purified IgG Fab fraction from dogs vaccinated with Leishmune® (4, 40 and 400 µg/ml) with 10⁶ procyclical promastigotes. Some minutes after the interaction, the intestines were added and co-incubated. Later the systems were individually washed and homogenized. The delivered Leishmania promastigotes were counted in a Neubauer chamber. The results represent the average and standard error of 2 experiments series with 7-10 membranes for each Fab fraction concentration.

The potential blocking of transmission by Leishmune vaccine in the visceral canine leishmaniasis investigated in the above described assays is summarized in the illustrations C and D. The analytic tests demonstrated expressive differences among growing FML concentrations, except between 4 and 40 µg/ml. Differences were not found between binding inhibition for *L. donovani* or *L. chagasi* thus indicating that antibodies induced by vaccinated dogs impede the binding of the procyclical promastigotes phases of both parasites to the medium intestine of the vectors, blocking and impeding the disease transmission.

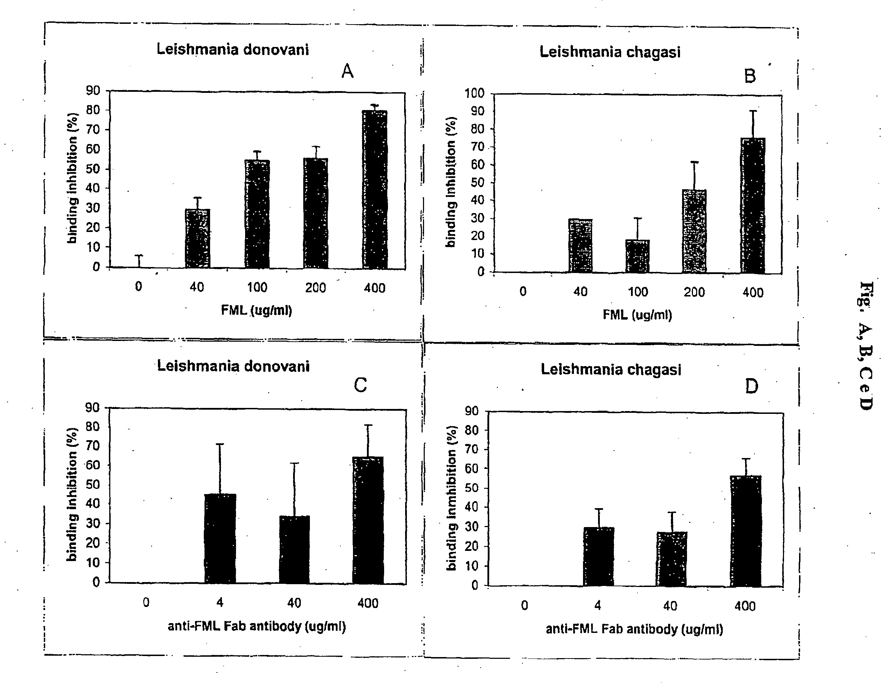

## Claims

1. A composition comprising Fucose Mannose Ligand (FML) and saponin, for use as a blocking vaccine against transmission of canine visceral Leishmaniasis, wherein administration of the composition to a canine subject produces an antibody in said subject in response to FML which inhibits the binding of Leishmaniasis donovani and chagasi procyclical promastigotes to the membrane of the medium intestine of Phlebotominae, thereby impeding the transmission of Leishmaniasis by Phlebotominae to humans and animals.

2. The composition of claim 1, wherein said composition further comprises suitable pharmacological vehicles.

3. The composition of claim 1, wherein the FML comprises a glycoprotein complex containing 29% of neutral sugar, 44% of protein, 11% of phosphate and hexoamines traces composed by protein bands with molecular weight ranging from 9 to 95 kDa, some protein bands having carbohydrates: 36 and 54 to 68 kDa, the FML glycidic portion presenting 10% of fucose and 47% of manose, 30% of glucose, 12% of galactose and xylose traces, aminoacids containing 10 to 20% of glutamic acid, alanine, aspartic acid and glycine, 5 to 10% of serine, histidine, lysine, leucine, threonine, proline and valine, and 2 to 5% of arginine, methionine, isoleucine, tyrosine and phenylalanine.

## Patentansprüche

1. Zusammensetzung, die Fucose-Mannose-Ligand (FML) und Saponin umfasst, zur Verwendung als blockierender Impfstoff gegen die Übertragung von caniner viszeraler Leishmaniose, wobei eine Verabreichung der Zusammensetzung an ein canines Subjekt einen Antikörper in dem Subjekt als Reaktion auf FML produziert, der die Bindung von prozyklischen Promastigoten von Leishmaniasis donovani und chagasi an die Membran des Mitteldarms von Phlebotominae inhibiert, wodurch die Übertragung von Leishmaniasis durch Phlebotominae auf Menschen und Tiere erschwert wird.

2. Zusammensetzung gemäß Anspruch 1, wobei die Zusammensetzung außerdem geeignete pharmakologische Vehikel umfasst.

3. Zusammensetzung gemäß Anspruch 1, wobei der FML einen Glykoproteinkomplex umfasst, enthaltend 29% neutralen Zucker, 44% Protein, 11% Phosphat und Hexoaminspuren, bestehend aus Proteinbanden mit einem Molekulargewicht im Bereich von 9 bis 95 kDa, wobei einige Proteinbanden Kohlenhydrate haben: 36 und 54 bis 68 kDa, wobei der glyzidische FML-Teil 10% Fucose und 47% Mannose, 30% Glucose, 12% Galactose und Xylosespuren aufweist, Aminosäuren 10 bis 20% Glutaminsäure, Alanin, Asparaginsäure und Glycin, 5 bis 10% Serin, Histidin, Lysin, Leucin, Threonin, Prolin und Valin und 2 bis 5% Arginin, Methionin, Isoleucin, Tyrosin und Phenylalanin enthalten.

## Revendications

1. Composition comprenant un ligand fucose-Mannose (FML) et de la saponine, pour être utilisée comme vaccin bloquant la transmission de la leishmaniose viscérale canine, dans laquelle l'administration de la composition à un sujet canin produit un anticorps chez ledit sujet en réponse au FML qui inhibe la liaison des promastigotes procycliques des leishmanioses donovani et chagasi à la membrane de l'intestin moyen des phlébotomes, empêchant ainsi la transmission de la leishmaniose par les phlébotomes aux humains et aux animaux.

2. Composition selon la revendication 1, ladite composition comprenant en outre des véhicules pharmacologiques adéquats.

3. Composition selon la revendication 1, dans laquelle le FML comprend un complexe de glycoprotéine contenant 29 % de sucre neutre, 44 % de protéine, 11 % de phosphate et de traces d'hexosamines composé de bandes protéiques ayant une masse moléculaire allant de 9 à 95 kDa, certaines bandes protéiques comportant des hydrates de carbone : 36 et 54 à 68 kDa, la portion glycidique du FML présentant 10 % de fucose et 47 % de mannose, 30 % de glucose, 12 % de galactose et des traces de xylose, des acides aminés contenant 10 à 20 % d'acide glutamique, d'alanine, d'acide aspartique et de glycine, 5 à 10 % de sérine, histidine, lysine, leucine, thréonine, proline et valine, et 2 à 5 % d'arginine, méthionine, isoleucine, tyrosine et phénylalanine.
